# EUROPÄISCHE PATENTSCHRIFT

(11) **EP 3 380 027 B1**
(45) Veröffentlichungstag und Bekanntmachung des Hinweises auf die Patenterteilung: **16.06.2021**
(21) Anmeldenummer: 16805339.5
(22) Anmeldetag: 23.11.2016
(51) Int. Cl.: A61B 17/84, A61B 17/064, A61L 31/02, A61L 31/14

(54) **BIOLOGISCH RESORBIERBARER FIXIERUNGSNAGEL**
BIORESORABLE FIXATION NAIL
CLOU DE FIXATION BIOLOGIQUEMENT RÉSORBABLE

(30) Priorität: 26.11.2015 DE 102015120514
(43) Veröffentlichungstag der Anmeldung: 03.10.2018
(73) Patentinhaber: Syntellix AG, 30159 Hannover (DE)
(72) Erfinder: SEITZ, Jan-Marten, 30159 Hannover (DE)
(74) Vertreter: Köllner, Malte
(86) Internationale Anmeldenummer: PCT/EP2016/078531
(87) Internationale Veröffentlichungsnummer: WO 2017/089381

(56) Entgegenhaltungen:
- EP-A1- 0 809 979
- WO-A2-2010/017959
- DE-A1- 19 509 966
- DE-A1-102009 025 511
- DE-A1-102011 082 210
- DE-U1- 29 513 342
- US-B1- 6 508 821

## Beschreibung

Die vorliegende Erfindung betrifft einen biologisch resorbierbaren Nagel zur Fixierung von Membranen, die zur Abdeckung von Knochendefektstellen eingesetzt werden oder zum Befestigen von natürlichen und künstlichen Knochenersatzteilen an Knochen.

Durch Zahnverlust, Parodontitis oder durch den Auflagedruck von Zahnprothesen kann ein Knochenabbau des Kieferknochens (Kieferabbau) erfolgen. Bei der Implantation von Zahnersatz kann es daher notwendig sein, zunächst einen Kieferaufbau vorzunehmen. Zahnimplantate sind in diesem Fall eine Art im Kieferknochen verankerte Stützpfeiler, die die Funktion einer künstlichen Zahnwurzel haben. Ist zu wenig Knochen vorhanden, kann eine dauerhafte stabile Integration des Implantats nicht gewährleistet werden.

Eine Knochenaugmentation (Knochenaufbau) kann bei nicht ausreichendem Knochenangebot zweizeitig, also im Vorfeld vor der eigentlichen Implantation, oder auch zeitgleich mit der eigentlichen Implantation des Zahnersatzes erfolgen. Beim Knochenaufbau wird entweder autologer Knochen vom selben Individuum, welches dem Patienten zunächst an geeigneter Stelle entnommen werden muss, oder Fremdmaterial verwendet. Das Fremdmaterial kann natürlichen Ursprungs sein, beispielsweise Spenderknochen, oder synthetisch hergestellt sein. Zum Aufbau und zur Regeneration des Kieferknochens wird der Defekt im Knochen mit dem autologen - oder Fremdmaterial aufgefüllt. Synthetische Knochenersatzstoffe, wie Hydroxylapatit und Calciumphosphat, werden meist in Form von Spänen oder Granulat verwendet. Autologes Knochenmaterial wird zumeist in Form von Bohrspänen eingesetzt.

Damit das Knochenaufbaumaterial nicht abwandern kann oder ausgewaschen wird und um zudem das Eindringen und Einwachsen von Weichteilzellen in die gefüllte Knochendefektstelle (langsamer regenerierendes Hartgewebe) zu verhindern, wird diese mit einer Abdeckmembran verschlossen (Prinzip der membrangeschützten Knochenregeneration - guided bone regeneration, GBR)

Abdeckmembranen sind aus dem Stand der Technik bekannt. Gängige Abdeckmembranen aus dem Stand der Technik werden zumeist mit Nägeln oder Schrauben aus Metallen wie Titan am Knochen befestigt, um ein Verrutschen zu verhindern. Ein bloßes Verklemmen oder Vernähen der Abdeckmembranen ist dabei nicht ausreichend. Die Zeit für die Ausheilung liegt im Allgemeinen zwischen 12 und 20 Wochen.

Die US 5,957,690 A offenbart eine Abdeckmembran aus dem flexiblen Kunststoffmaterial Polytetraflourethylen (PTFE).

In der DE 43 02 709 C1 wird eine Abdeckmembran mit einer Versteifungslage beschrieben. Diese Membran wird mittels Befestigungsnägeln am Kieferknochen fixiert.

Eine aus unelastisch spannungsfreien und bleibend verformbaren Titan geformte Abdeckfolie sowie ein entsprechender Nagel zur Lagefixierung sind in der EP 0 809 979 A1 offenbart.

Auch sind bereits vom Körper resorbierbare Abdeckmembrane aus Kollagenfasern oder ähnlichen Materialien bekannt.

Nicht resorbierbare Fixierungsmittel, wie Titannägel oder Schrauben, oder auch nicht resorbiere Abdeckmembrane haben den Nachteil, dass nach erfolgtem Einsatz des Knochenersatzmaterials und der Ausheilung der Wunde die nicht resorbierbaren Befestigungsmittel und Abdeckmembrane durch einen chirurgischen Eingriff wieder entfernt werden müssen, bevor das eigentliche Zahnimplantat eingesetzt werden kann. Dies stellt eine erhebliche Irritation des betroffenen Bereichs dar.

Bei Knochendefekten an anderen Körperstellen werden natürliche oder künstliche Knochenersatzteile am angrenzenden Knochen mittels unterschiedlicher Fixierungshilfen befestigt. Auch hier müssen nicht resorbierbare Fixierungsmittel zumeist wieder operativ aus dem Körper entfernt werden, sobald der Knochen geheilt ist.

Die deutsche Gebrauchsmusterschrift DE 295 13 342 U1 offenbart einen resorbierbaren Fixierungsnagel gemäß dem Oberbegriff der unabhängigen Ansprüche 1 und 6.

Aufgabe der vorliegenden Erfindung war es daher, ein alternatives Befestigungsmittel bereit zu stellen, welches die bekannten Nachteile aus dem Stand der Technik vermeidet.

Gelöst wird die Aufgabe durch einen Fixierungsnagel gemäß den unabhängigen Ansprüchen 1 oder 6. Wird in der Kieferchirurgie eine abbaubare Abdeckmembran zusammen mit dem biokompartiblem und biokorrodierbaren Fixierungsnagel verwendet, so erfolgt im Verlaufe der Wundheilung - die in der Regel 12 - 20 Wochen dauert- eine vollständige Resorption sowohl der wundabdeckenden Membran als auch der Fixierungshilfe, nämlich der erfindungsgemäßen Fixierungsnägel. Am Ende liegt ein verstärkter Knochen vor, der von gesundem Zahnfleisch umgeben ist. Der Chirurg kann ein Zahnimplantat setzen, ohne dabei zuerst Rückstände der Membran, Hilfsmittel oder Titanstifte entfernen zu müssen.

Auch bei Knochendefekten an anderen Körperstellen entfällt die operative Entfernung nicht resorbierbarer Fixierungsmittel durch Verwendung des erfindungsgemäßen Fixierungsnagels aus Magnesium.

Der Vorteil gegenüber vergleichbaren Fixierstiften oder Fixierungsnägeln aus Titan oder Edelstahl liegt darin, dass die erfindungsgemäßen Magnesiumnägel zusammen mit dem meist ebenfalls abbaubaren Abdeck-Gewebe, wie Kollagen Membrane, im Lauf der Heilung vollständig vom Körper resorbiert werden und nicht durch erneuten chirurgischen Eingriff vor oder während des Setzens des eigentlichen Zahn-Implantats entfernt werden müssen. Magnesium hat zudem einen den Knochenaufbau fördernden Effekt (Switzer, E.N., Resorbierbares metallisches Osteosynthesematerial. Untersuchungen zum Resorptionsverhalten im Meerschweinchen-Modell, Dissertation, Tierärztliche Hochschule Hannover, 2005). Auch ist bekannt, dass die bei der Resorption frei werdenden Magnesium-Ionen den Knochenaufbau stimulieren (WO 2015/133963 A).

Aus der DE 10 2011 082 210 A1 sind bereits medizinische Implantate in Form einer Knochenschraube, eines Knochennagels oder eines Knochenstiftes bekannt, die aus einer resorbierbaren Magnesiumlegierung bestehen. Allerdings treten bei deren fester Fixierung durch Eindrücken oder Einschlagen insbesondere im Bereich der Unter- oder Oberkieferprothese Probleme auf. Deshalb muss der Nagel einerseits eine ausreichende mechanische Stabilität aufweisen, andererseits muss verhindert werden, dass der Nagel oder die Schraube zu tief in das Knochengewebe eindringt.

Diese Probleme können gelöst werden, wenn der Nagel oder die Schraube einerseits einen genügend breiten Kopf aufweisen und andererseits mit einem langen und dicken Stift versehen sind. Vorteilhaft ist es außerdem, **wenn der Nagel mit Widerhaken versehen ist, die eine sichere Verankerung des Nagels im Knochengewebe gewährleisten.**

In einer bevorzugten Ausführungsform beträgt der Durchmesser des Nagelkopfes 1,5 bis 4,0 mm, die Länge des spitzen Nagelstifts beträgt 0,75 bis 8,0 mm, und die Dicke des Nagelkopfes 0,4 bis 1,0 mm und die Dicke des Nagelstifts 0,22 bis 2,0 mm. Der Nagelkopf ist dabei vorzugsweise in der Form eines Reißbrettstiftes gestaltet.

Weiterhin ist es bevorzugt, dass der Durchmesser des Nagelkopfes vorzugsweise 2,5 mm beträgt, dass die Länge des spitzen Nagelstifts vorzugsweise 2,4 mm beträgt, dass die Dicke des Nagelkopfes vorzugsweise 0,6 mm beträgt und dass die Dicke des Nagelstifts maximal 0,8 mm beträgt.

Die feste Fixierung der Abdeckmembran wird durch Eindrücken oder Einschlagen des erfindungsgemäßen Magnesiumnagels in den Kieferknochen erreicht. Die Fixierungsnägel werden an Transplantationsorten eingesetzt, die schwer zugänglich sind etwa im Bereich von Unter- und Oberkiefer. Dem Chirurg dient dabei ein dünnes bleistiftförmiges Applikationsgerät, welches den Kopf des Nagels mit Klemmen umfasst und das Positionieren an geeigneter Stelle ermöglicht. Anschließend wird der Nagel mit Hilfe des Applikators eingedrückt oder mit einem chirurgischen Hammer in den Knochen eingeschlagen. Dafür muss der Nagelstift eine gewisse mechanische Stabilität aufweisen, die in erster Linie durch die Materialstärke des Stifts und die chemische Zusammensetzung und den Herstellprozess der Legierung gegeben ist. Durch den breiten Kopf des Nagels wird verhindert, dass der Nagel vollständig durch die Abdeckmembran hindurch getrieben wird.

Vorteilhaft für die Verankerung des Nagels ist es, wenn im unteren Bereich des Nagelstifts, etwa unterhalb der Mitte, ein oder zwei Widerhaken vorgesehen sind. Sie erhöhen die Festigkeit der Verankerung und erschweren das Ausziehen des Nagels. In einer Ausgestaltungsform der Erfindung weist daher der Nagelstift mindestens einen Widerhaken auf.

Es ist weiterhin bevorzugt, dass der Nagelstift einen oder zwei Widerhaken im Bereich der unteren Hälfte der Stiftlänge aufweist.

Der oder die Widerhaken werden vorzugsweise durch radiales Abdrehen des Nagelstifts um 0,1 bis 0,4 mm erzeugt.

Der Magnesiumnagel muss für eine bestimmte Zeit eine feste Fixierung der das eingebrachte Knochenmaterial abdeckenden Membran oder des transplantierten Knochenstückes gewährleisten. Die Geschwindigkeit der Resorption des Magnesiumnagels wird in erster Linie durch die chemische Zusammensetzung der Legierung und deren Herstellung bestimmt. Durch Zusätze bestimmter Legierungselemente, besonders von Zink, Zirkonium und Seltenen Erd-Metallen (Legierungen aus Yttrium, Lanthan, Neodym, Dysprosium und anderen) wird sowohl die mechanische Stabilität der Magnesiumstifte als auch deren Resorptionsrate beeinflusst. Die angegebenen minimalen und maximalen Grenzwerte für die Zusammensetzung dieser Legierungen sind so gewählt, dass eine Resorption im Zeitrahmen der Ausheilung der Knochen-Transplantation bzw. des Knochenaufbaus vollständig gewährleistet ist.

Die physiologische Unbedenklichkeit der Magnesiumnägel wird in erster Linie durch den Gehalt an unvermeidbaren Verunreinigungen bestimmt. Es sind daher maximale Mengen an physiologisch bedenklichen Spurenelementen angegeben.

Die physiologische Unbedenklichkeit von Magnesium als biokorrodierbares Metall ist aus anderen Studien bekannt (Schrenk, Sebastian, "Abbauverhalten degradierbarer Magnesiumlegierungen in körperähnlichen Flüssigkeiten", Dissertation, Medizinische Fakultät der Friedrich-Alexander-Universität Erlangen-Nürnberg 2011).

Vorzugsweise besteht der Fixiernagel aus einer biokorrodierbaren Magnesiumbasislegierung, die zusammengesetzt ist aus Magnesium, Yttrium, Zirkonium und sonstigen Selten-Erd-Metallen.

Gemäß einem weiteren Aspekt der vorliegenden Erfindung besteht der Fixierungsnagel aus einer biokorrodierbaren Magnesiumlegierung, die zusammengesetzt ist aus metallischem Magnesium von mind. 80 Gew. %, einem Anteil von Zink von 0,1 bis 3,0 Gew. %, einem Anteil von Zirkonium von 0,1 bis 3,0 Gew. %, einem Anteil von Selten-Erdmetallen von insgesamt 0,1 bis 10,0 Gew. % und physiologisch unerwünschten Verunreinigungen.

Es ist bevorzugt, dass von dem Anteil von Selten-Erdmetallen von insgesamt 0,1 bis 10,0 Gew. % der Yttriumanteil von 1,5 bis 5 Gew.-% beträgt.

Erfindungsgemäß ist vorgesehen, dass die Magnesiumbasislegierung unter 0,01 Gew.-% Aluminium, unter 0,20 Gew.-% Zink, unter 0,15 Gew.-% Mangan, unter 0,20 Gew.-% Lithium, unter 0,01 Gew.-% Silizium, unter 0,01 Gew.-% Eisen, unter 0,03 Gew.-% Kupfer und unter 0,005 Gew.-% Nickel enthält.

Anstelle der Widerhaken kann es genügen, die Spitze des Nagels harpunenförmig zu gestalten oder lanzettförmig auszuführen.

Es ist daher weiterhin bevorzugt dass die Spitze des Nagelstifts harpunenförmig ausgebildet ist

Auch ist es, bevorzugt dass die Spitze des Nagelstifts lanzenförmig ausgebildet ist.

In einer weiteren Ausgestaltungsform ist der Nagelkopf haubenförmig gestaltet.

Der Nagelstift kann zudem über seine gesamte Länge oder einen Teil seiner Länge mit Rillen versehen werden, um insbesondere die anfängliche Verankerung im Knochen zu verbessern und ein ungewolltes Herausziehen zu verhindern.

In einer weiteren bevorzugten Ausführungsform weist der Nagelstift daher mindestens eine, vorzugsweise 2 bis 5 rillenförmige Einschnürungen von 0,1 bis 0,2 mm im Bereich des unteren Drittels des Fixierstiftes auf.

In einer Ausführungsform weist der Nagelstift mindestens eine Ausbuchtung von 0,1 bis 0,3 mm im Bereich des unteren Drittels des Fixierstiftes auf.

Weitere Vorteile ergeben sich aus einer Verwendung des erfindungsgemäßen Fixierungsnagels zum temporären Befestigen stabilisierender Gewebe bei Knochenaugmentationen. Der Einsatz des erfindungsgemäßen Fixierungsnagels beschränkt sich daher nicht nur auf den kieferchirurgischen Bereich, sondern kann zur Fixierung von natürlichen oder künstlichen stabilisierendem Gewebe sowie natürlichen und künstlichem Knochenmaterial am körpereigenen Knochen verwendet werden.

Zudem ist eine Verwendung des erfindungsgemäßen Fixierungsnagels zum temporären Befestigen von resorbierbaren und nicht-resorbieren Abdeckmembranen bei Zahnimplantationen vorgesehen. Vorzugsweise handelt es sich dabei um eine Verwendung zum temporären Befestigen von kollagen-haltigen und knochenaufbauendem Gewebe.

Die vorliegende Erfindung wird anhand folgender Figuren näher erläutert.
Fig. 1 zeigt den erfindungsgemäßen Fixierungsnagel mit einer harpunenförmigen Spitze des Nagelstifts.
Fig. 2 zeigt den erfindungsgemäßen Fixierungsnagel mit einer lanzenförmigen Spitze des Nagelstifts.
Fig. 3 zeigt den erfindungsgemäßen Fixierungsnagel mit Rillen im Nagelstift.
Fig. 4 zeigt den erfindungsgemäßen Fixierungsnagel mit einem haubenförmigen Nagelkopf.
Fig. 5 zeigt eine Detailzeichnung eines erfindungsgemäßen Magnesiumnagels zur Verwendung in der Zahnmedizin.
Fig. 6 zeigt eine dreidimensionale Darstellung des erfindungsgemäßen Magnesiumnagels nach Fig.5.

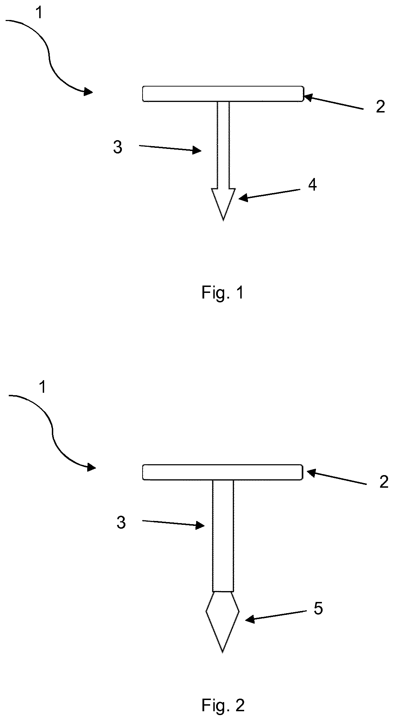

Die Figuren 1 bis 6 zeigen jeweils eine Ausführungsform des erfindungsgemäßen Fixierungsnagels (1). Dieser besteht aus einem Nagelkopf (2) und einem Nagel-stift (3). Die Spitze des Nagelstifts (3) kann dabei harpunenförmig (4) (Fig. 1) oder lanzenförmig (5) (Fig. 2) gestaltet sein, um eine feste Verankerung im Knochen zu gewährleisten.

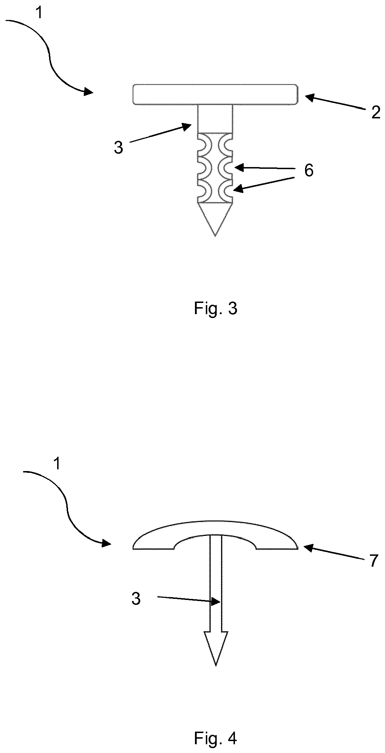

Auch kann der Nagelstift (3) über die gesamte Länge oder einen Teil seiner Länge mit Rillen (6) versehen werden (Fig. 3), um insbesondere die anfängliche Verankerung im Knochen zu verbessern und ein ungewolltes Herausziehen zu verhindern.

Das "Durchreißen" des Nagelkopfes durch die verankerte Abdeckmembran wird durch den im Verhältnis zur Länge des Stifts breiten Kopf des Nagels verhindert. Der Nagelkopf muss ebenfalls eine ausreichende mechanische Stabilität besitzen und darf sich beim Eintreiben des Nagels nicht verbiegen. Der Nagelkopf kann haubenförmig (7) gestaltet sein (Fig. 4).

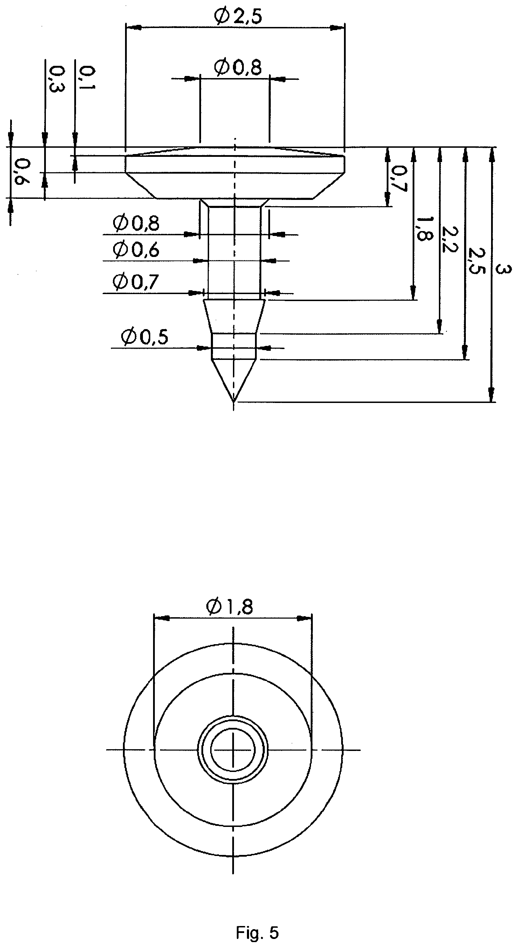

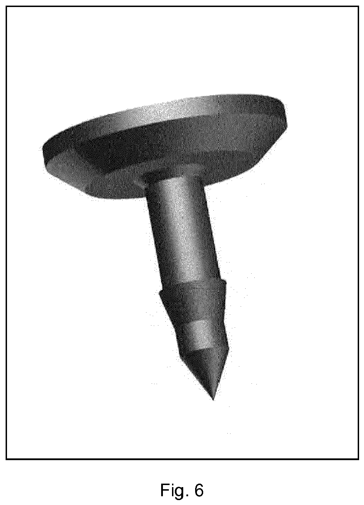

Die Fig. 5 und 6 zeigen eine typische in der Klinik erprobte Ausführung des erfindungsgemäßen Magnesiumnagels. Eine besonders geeignete Ausführung der Erfindung ist in der Fig. 5 dargestellt. Der dort in einer CAD Zeichnung skizzierte Magnesiumnagel ist gekennzeichnet durch einen Nagelkopf von 2,5 mm Durchmesser und einer Materialdicke von 0,7 mm. Der Nagelstift ist gekennzeichnet durch eine Länge von 2,4 mm und eine Materialstärke von 0,7 mm. Der Nagelstift hat etwa in der Mitte eine Ausfräsung von 0,1 mm zur Formung eines Widerhakens. Der Nagelkopf hat am Rand Fasen, die es erleichtern, den Pin mit dem Applikator zu greifen. Die Fig. 6 zeigt den erfindungsgemäßen Magnesiumnagel in einer dreidimensionalen Ansicht. Dieser Magnesiumnagel ist besonders zur Verwendung bei der Knochenaugmentation im Rahmen von Zahnimplantationen geeignet.

Bei Verwendung der erfindungsgemäßen Magnesiumnägel, insbesondere eines Nagels entsprechend Fig. 5 und Fig. 6 werden alle klinischen Forderungen erfüllt. Die physiologische Unbedenklichkeit von Magnesium als biokorrodierbares Metall ist aus anderen Studien bekannt (Schrenk, Sebastian, "Abbauverhalten degradierbarer Magnesiumlegierungen in körperähnlichen Flüssigkeiten", Dissertation, Medizinische Fakultät der Friedrich-Alexander-Universität Erlangen-Nürnberg 2011.).

### Bezugszeichenliste

1. Fixierungsnagel
2. Nagelkopf
3. Nagelstift
4. harpunenförmige Spitze des Nagelstifts
5. lanzettförmige Spitze des Nagelstifts
6. Rillen
7. Nagelkopf haubenförmig

## Patentansprüche

1. Fixierungsnagel (1) zum temporären Befestigen von transplantierbarem Gewebe, Knochenteilen und Knochenersatzsubstanzen mittels Abdeckung oder zum Befestigen von natürlichen und künstlichen Knochenersatzteilen an Knochen,
wobei
der Fixierungsnagel (1) einen runden und flachen Nagelkopf (2) von 0,50 bis 6,0 mm Durchmesser aufweist,
die Dicke des Nagelkopfes (2) 0,10 bis 2,0 mm beträgt,
der Fixierungsnagel (1) einen mit einer scharfen Spitze ausgestalteten Nagel-Stift (3) aufweist, dessen Länge zwischen dem 0,5-fachen und dem 2-fachen des Durchmessers des Nagelkopfes (2) beträgt und dessen Stiftdicke zwischen dem 0,15-fachen und dem 0,5-fachen des Durchmessers des Nagelkopfes (2) beträgt, dadurch charakterisiert, dass
der Fixierungsnagel (1) aus einer biokompatiblen, biokorrodierbaren Magnesiumlegierung besteht, die mindestens 90 Gew.% metallisches Magnesium enthält,
wobei
die Magnesiumlegierung weniger als 0,01 Gew.% Aluminium, weniger als 0,20 Gew.% Zink, weniger als 0,15 Gew.% Mangan, weniger als 0,20 Gew.% Lithium, weniger als 0,01 Gew.% Silizium, weniger als 0,03 Gew.% Kupfer, weniger als 0,01 Gew.% Eisen und weniger als 0,005 Gew.% Nickel als physiologisch unerwünschte Verunreinigungen enthält.

2. Fixierungsnagel (1) nach Anspruch 1, **dadurch gekennzeichnet, dass** der Durchmesser des Nagelkopfes (2) 1,5 bis 4,0 mm beträgt, die Länge des spitzen Nagelstifts (3) 0,75 bis 8,0 mm beträgt, die Dicke des Nagelkopfes (2) 0,4 bis 1,0 mm beträgt und die Dicke des Nagelstifts (3) 0,22 bis 2,0 mm beträgt.

3. Fixierungsnagel (1) nach Anspruch 1, **dadurch gekennzeichnet, dass** der Durchmesser des Nagelkopfes (2) vorzugsweise 2,5 mm beträgt, die Länge des spitzen Nagelstifts (3) vorzugsweise 2,4 mm beträgt, die Dicke des Nagelkopfes (2) vorzugsweise 0,6 mm beträgt und die die Dicke des Nagelstiftes (3) maximal 0,8 mm beträgt.

4. Fixierungsnagel (1) nach den Ansprüchen 1 oder 2, **dadurch gekennzeichnet, dass** der Nagelstift (3) mindestens einen Widerhaken aufweist.

5. Fixierungsnagel (1) nach einem der vorherigen Ansprüche, **dadurch gekennzeichnet, dass** der Nagelstift (3) einen oder zwei Widerhaken im Bereich der unteren Hälfte der Stiftlänge aufweist.

6. Fixierungsnagel (1) zum temporären Befestigen von transplantierbarem Gewebe, Knochenteilen und Knochenersatzsubstanzen mittels Abdeckung oder zum Befestigen von natürlichen und künstlichen Knochenersatzteilen an Knochen, wobei
der Fixierungsnagel (1) einen runden und flachen Nagelkopf (2) von 0,5 bis 6,0 mm Durchmesser aufweist,
die Dicke des Nagelkopfes (2) 0,1 bis 2,0 mm beträgt,
der Fixierungsnagel (1) einen mit einer scharfen Spitze ausgestalteten Nagel-Stift (3) aufweist, dessen Länge zwischen dem 0,5-fachen und dem 2-fachen des Durchmessers des Nagelkopfes (2) beträgt und dessen Stiftdicke zwischen dem 0,15-fachen und dem 0,5-fachen des Durchmessers des Nagelkopfes (2) beträgt, dadurch charakterisiert, dass
der Fixierungsnagel aus einer biokompatiblen, biokorrodierbaren Magnesiumlegierung besteht, die
mindestens 80 Gew. % metallisches Magnesium, sowie 0,1 bis 3,0 Gew. % Zink 0,1 bis 3,0 Gew. % Zirkonium, sowie 0,1 bis 10,0 Gew. % Seltenerdmetalle enthält, wobei
die Magnesiumlegierung weniger als 0,01 Gew.% Aluminium,
weniger als 0,15 Gew.% Mangan, weniger als 0,20 Gew.% Lithium, weniger als 0,01 Gew.% Silizium, weniger als 0,03 Gew.% Kupfer, weniger als 0,01 Gew.% Eisen und weniger als 0,005 Gew.% Nickel als physiologisch unerwünschte Verunreinigungen enthält.

7. Fixierungsnagel (1) nach einem der vorherigen Ansprüche, **dadurch gekennzeichnet, dass** der Nagelstift (3) mindestens eine Ausbuchtung von 0,1 bis 0,3 mm im Bereich des unteren Drittels des Fixierstiftes aufweist.

8. Fixierungsnagel (1) nach einem der vorherigen Ansprüche, **dadurch gekennzeichnet, dass** der Nagelstift (3) mindestens eine, vorzugsweise 2 bis 5 rillenförmige Einschnürungen oder rillenförmige Ausfräsungen (6) von 0,1 bis 0,2 mm aufweist.

9. Fixierungsnagel (1) nach einem der vorherigen Ansprüche, **dadurch gekennzeichnet, dass** der Nagelkopf (2) haubenförmig (7) gestaltet ist.

10. Fixierungsnagel (1) nach einem der vorherigen Ansprüche, **dadurch gekennzeichnet, dass** die Spitze des Nagelstifts (3) harpunenförmig (4) ausgebildet ist

11. Fixierungsnagel (1) nach einem der Ansprüche 1-9, **dadurch gekennzeichnet, dass** die Spitze des Nagelstifts (3) lanzenförmig (5) ausgebildet ist.

## Claims

1. Fixing nail (1) for temporary fixation of transplantable tissue, bone parts and bone substitutes by means of covering or for fixation of natural and artificial bone substitutes to bone,
wherein
the fixing nail (1) has a round and flat nail head (2) of 0.50 to 6.0 mm diameter,
the thickness of nail head (2) is 0.10 to 2.0 mm,
the fixing nail (1) has a nail pin (3) comprising a sharp tip, wherein the length of the pin is between 0.5 times and 2 times the diameter of nail head (2), and the pin thickness is between 0.15 times and 0.5 times the diameter of the nail head (2), **characterized in that**
fixing nail (1) consists of a biocompatible, biocorrodible magnesium alloy containing at least 90% by weight of metallic magnesium,
wherein
the magnesium alloy contains less than 0.01 wt.% aluminum, less than 0.20 wt.% zinc, less than 0.15 wt.% manganese, less than 0.20 wt.% lithium, less than 0.01 wt.% silicon, less than 0.03 wt.% copper, less than 0.01 wt.% iron, and less than 0.005 wt.% nickel as physiologically undesirable impurities.

2. Fixing nail (1) according to claim 1, **characterized in that**
the diameter of nail head (2) is 1.5 to 4.0 mm, the length of nail pin (3) is 0.75 to 8.0 mm, the thickness of nail head (2) is 0.4 to 1.0 mm, and the thickness of nail pin (3) is 0.22 to 2.0 mm.

3. Fixing nail (1) according to claim 1, **characterized in that**
the diameter of nail head (2) is preferably 2.5 mm, the length of nail pin (3) is preferably 2.4 mm, the thickness of nail head (2) is preferably 0.6 mm and the thickness of nail pin (3) is at most 0.8 mm.

4. Fixing nail (1) according to claims 1 or 2, **characterized in that** the nail pin (3) has at least one barb.

5. Fixing nail (1) according to one of the previous claims, **characterized in that** nail pin (3) has one or two barbs in the region of the lower half of the pin length.

6. Fixing nail (1) for temporary fixation of transplantable tissue, bone parts and bone substitutes by means of covering or for fixation of natural and artificial bone substitutes to bone,
wherein
the fixing nail (1) has a round and flat nail head (2) of 0.5 to 6.0 mm diameter,
the thickness of nail head (2) is 0.1 to 2.0 mm,
the fixing nail (1) has a nail pin (3) comprising a sharp tip, wherein the length of the pin is between 0.5 times and 2 times the diameter of nail head (2), and the pin thickness is between 0.15 times and 0.5 times the diameter of nail head (2), **characterized in that**
fixing nail (1) consists of a biocompatible, biocorrodible magnesium alloy containing at least 80 wt.% metallic magnesium, as well as 0.1 to 3.0 wt.% zinc, 0.1 to 3.0 wt.% zirconium, and 0.1 to 10.0 wt.% rare earth metals,
wherein
the magnesium alloy contains less than 0.01 wt.% aluminum, less than 0.15 wt.% manganese, less than 0.20 wt.% lithium, less than 0.01 wt.% silicon, less than 0.03 wt.% copper, less than 0.01 wt.% iron, and less than 0.005 wt.% nickel as physiologically undesirable impurities.

7. Fixing nail (1) according to one of the previous claims, **characterized in that** nail pin (3) has at least one bulge of 0.1 to 0.3 mm in the region of the lower third of the pin.

8. Fixing nail (1) according to one of the previous claims, **characterized in that** nail pin (3) has at least one, preferably 2 to 5, groove-shaped constrictions or groove-shaped milled-out portions (6) of 0.1 to 0.2 mm.

9. Fixing nail (1) according to one of the previous claims, **characterized in that** nail head (2) is hood-shaped (7).

10. Fixing nail (1) according to one of the previous claims, **characterized in that** the tip of nail pin (3) is harpoon-shaped (4).

11. Fixing nail (1) according to one of claims 1-9, **characterized in that** the tip of nail pin (3) is lance-shaped (5).

## Revendications

1. Clou de fixation (1) pour la fixation temporaire de tissus, de parties d'os et de substituts osseux transplantables au moyen d'un recouvrement ou pour la fixation de substituts osseux naturels et artificiels à l'os,
- dans lequel le clou de fixation (1) a une tête de clou ronde et plate (2) de 0,50 à 6,0 mm de diamètre,
- l'épaisseur de la tête de clou (2) est de 0,10 à 2,0 mm,
- le clou de fixation (1) comporte une broche de clou (3) formée d'une pointe acérée, dont la longueur est comprise entre 0,5 fois et 2 fois le diamètre de la tête de clou (2) et dont l'épaisseur de la broche est comprise entre 0,15 fois et 0,5 fois le diamètre de la tête de clou (2),
**caractérisé en ce que**
- le clou de fixation (1) est constitué d'un alliage de magnésium biocompatible et biocorrodible contenant au moins 90 % en poids de magnésium métallique,
- dans lequel l'alliage de magnésium contient moins de 0,01 % en poids d'aluminium, moins de 0,20 % en poids de zinc, moins de 0,15 % en poids de manganèse, moins de 0,20 % en poids de lithium, moins de 0,01 % en poids de silicium, moins de 0,03 % en poids de cuivre, moins de 0,01 % en poids de fer et moins de 0,005 % en poids de nickel en tant qu'impuretés physiologiquement non souhaitées.

2. Clou de fixation (1) selon la revendication 1, **caractérisé en ce que**
le diamètre de la tête de clou (2) est de 1,5 à 4,0 mm, la longueur de la broche de clou (3) acérée est de 0,75 à 8,0 mm, l'épaisseur de la tête de clou (2) est de 0,4 à 1,0 mm, et l'épaisseur de la broche de clou (3) est de 0,22 à 2,0 mm.

3. Clou de fixation (1) selon la revendication 1, **caractérisé en ce que**
le diamètre de la tête de clou (2) est de préférence de 2,5 mm, la longueur de la broche de clou (3) acérée est de préférence de 2,4 mm, l'épaisseur de la tête de clou (2) est de préférence de 0,6 mm et l'épaisseur de la broche de clou (3) est au maximum de 0,8 mm.

4. Clou de fixation (1) selon les revendications 1 ou 2, **caractérisé en ce que** la broche de clou (3) présente au moins un barbillon.

5. Clou de fixation (1) selon l'une quelconque des revendications précédentes, **caractérisé en ce que** la broche de clou (3) présente un ou deux barbillons dans la région de la moitié inférieure de la longueur de la broche.

6. Clou de fixation (1) pour la fixation temporaire de tissus, de parties d'os et de substituts osseux transplantables au moyen d'un recouvrement ou pour la fixation de substituts osseux naturels et artificiels à l'os, dans lequel
- le clou de fixation (1) a une tête de clou ronde et plate (2) de 0,5 à 6,0 mm de diamètre,
- l'épaisseur de la tête de clou (2) est de 0,1 à 2,0 mm,
- le clou de fixation (1) présente une broche de clou (3) conçue avec une pointe acérée, dont la longueur est comprise entre 0,5 fois et 2 fois le diamètre de la tête de clou (2) et dont l'épaisseur de la broche est comprise entre 0,15 fois et 0,5 fois le diamètre de la tête de clou (2),
**caractérisé en ce que**
- le clou de fixation est constitué d'un alliage de magnésium biocompatible et biocorrosif qui contient au moins 80 % en poids de magnésium métallique, ainsi que 0,1 à 3,0 % en poids de zinc, 0,1 à 3,0 % en poids de zirconium, ainsi que 0,1 à 10,0 % en poids de métaux des terres rares,
- ledit alliage de magnésium contenant moins de 0,01% en poids d'aluminium, moins de 0,15% en poids de manganèse, moins de 0,20% en poids de lithium, moins de 0,01% en poids de silicium, moins de 0,03% en poids de cuivre, moins de 0,01% en poids de fer, et moins de 0,005% en poids de nickel en tant qu'impuretés physiologiquement indésirables.

7. Clou de fixation (1) selon l'une quelconque des revendications précédentes, **caractérisé en ce que** la broche (3) du clou présente au moins un renflement de 0,1 à 0,3 mm dans la région du tiers inférieur de la broche de fixation.

8. Clou de fixation (1) selon l'une quelconque des revendications précédentes, **caractérisé en ce que** la broche de clou (3) présente au moins un, de préférence 2 à 5, rétrécissements en forme de rainure ou des fraisures en forme de rainure (6) de 0,1 à 0,2 mm.

9. Clou de fixation (1) selon l'une quelconque des revendications précédentes, **caractérisé en ce que** la tête du clou (2) est en forme de capot (7).

10. Clou de fixation (1) selon l'une des revendications précédentes, **caractérisé en ce que** la pointe de la broche du clou (3) est en forme de harpon (4).

11. Clou de fixation (1) selon l'une quelconque des revendications 1 à 9, **caractérisé en ce que** la pointe de la broche de clou (3) est en forme de lance (5).
